# EUROPEAN PATENT APPLICATION

(11) **EP 0 611 574 A1**
(43) Date of publication of application: **24.08.1994**
(21) Application number: 93914990.2
(22) Date of filing: 12.07.1993
(51) Int. Cl.: A61K 49/00

(54) **NMR AGENT**

(30) Priority: 13.07.1992 JP 185122/92
(71) Applicant: ARAI, Toshiyuki, Kyoto-shi, Kyoto 606 (JP); MORI, Kenjiro, Sakyo-ku, Kyoto-shi, Kyoto 606 (JP)
(72) Inventor: ARAI, Toshiyuki, Kyoto-shi, Kyoto 606 (JP); MORI, Kenjiro, Sakyo-ku, Kyoto-shi, Kyoto 606 (JP)
(74) Representative: Stachow, E.-W., Prof. Dr.
(86) International application number: JP9300962
(87) International publication number: WO9401141

(57) **Abstract**

This invention aims at reducing a cost by avoiding the use of concentrated ¹⁷O and to make it possible to obtain accurate data on metabolism without undergoing any change in a signal intensity due to the influences of the blood circulated later even when a large amount of an NMR agent is dosed. The NMR agent of this invention is characterized in that it contains oxygen at least in such a concentration as to be necessary for maintaining life and the content of an oxygen isotope ¹⁷O in the oxygen is set to be less than a natural occurrence. It can be produced extremely economically by concentrating ¹⁶O, for example, and even when a large quantity of the agent is dosed, it mixes with the blood having a high H₂¹⁷O concentration during circulation inside the body, and the H₂¹⁷O content in the blood as a whole does not change much. Accordingly, the circulating blood does not greatly affect the signal intensity.

## Description

### (Technical Field)

The present invention concerns a nuclear magnetic resonance agent which is used upon causing hydrogen H in H₂¹⁶O present in a living body to nuclear magnetic resonance and detecting change with time of resonance signals thereof.

### (Background Art)

Nuclear magnetic resonance tomography (NMR-CT) used for a nuclear magnetic resonance diagnostic method can conduct tomograph for an inclined surface at an optional angle more easily as compared with conventional X-ray CT scanners, further does not suffer from hindrance for imaging by bones or air and has extremely high safety with no worry of causing X-ray exposure.

Then, tomographic images are formed, for instance, by irradiating sinusoidal signals depending on the resonance frequency of hydrogen atom H to a living body and detecting the state of the proton of hydrogen H present as a water content H₂O in the living body by detecting resonance signals.

By the way, oxygen includes, in addition to ordinary oxygen ¹⁶O, radioisotope ¹⁵O and stable isotope ¹⁷O and ¹⁸O, in which the stable isotope ¹⁷O has quite the same chemical property as that of ordinary oxygen ¹⁶O and, accordingly, it gives no undesired effects at all when it is intaken into the living body and is served to the metabolism in the same manner as ordinary oxygen.

In view of the above, the present inventor, et al have proposed a nuclear magnetic resonance agent capable of obtaining information on the metabolism by inhaling oxygen isotope ¹⁷O in the form of an oxygen gas to produce H₂¹⁷O by the metabolic function and detecting the intensity change of resonance signals by the effect thereof (refer to Japanese Patent Laid-Open Hei 3-167843).

According to this, since H₂¹⁷O is produced and increased by the metabolism of living cells merely by inhaling the nuclear magnetic resonance agent, if NMR-CT detects, for example, the proton of H₂O, since the relaxation time for the proton is shortened for a portion in which the metabolism is vigorous to produce a great amount of H₂¹⁷O, the signal intensity is weakened, whereas the signal intensity does not change for a portion in which the metabolism is failed to produce no H₂¹⁷O such as a focal portion.

Accordingly, it can be judged simply where a focal portion is present by detecting the change of the signal intensity or observing the change of the brightness of tomograph images based on the resonance signals.

However, in a case of finding the focal portion by the effect of H₂¹⁷O, while it is judged that a portion in which the signal intensity lowers or a portion which is darkened in tomograph is normal, when a great amount of ¹⁷O is administrated, concentration of H₂¹⁷O in blood is increased, which is circulated by a blood flow again to a measuring site, and the signal intensity changes under the effect of the circulated H₂¹⁷O, to bring about a problem that the focal portion and the lowering degree of the function thereof can not be recognized accurately.

That is, although the signal intensity ought to cause no change in the focal portion even when the nuclear magnetic resonance agent is administrated since H₂¹⁷O is not produced, the signal intensity lowers by the circulation of blood at high H₂¹⁷O concentration in the focal portion, which sometimes leads to erroneous judgment.

Further, when it is intended to find a focal portion by the effect of H₂¹⁷O, oxygen obtained by concentrating ¹⁷O has to be used. However, since the oxygen isotope ¹⁷O is contained only by 0.037% in usual oxygen, a production cost concentrating the same is extremely expensive and it can not be used at a reduced cost.

In view of the above, it is a subject of the present invention to remarkably reduce the production cost, as well as obtain an accurate information on the metabolic function based on the change of the signal intensity without changing of the signal intensity under the effect of blood circulated after great amount of administration.

### (Disclosure of the Invention)

For attaining the subject, the present invention has a feature in that oxygen at a concentration necessary for sustaining life is incorporated in an inhalation gas and the ratio of oxygen isotope ¹⁷O contained in the oxygen is selected to less than the naturally existing ratio.

According to this, since the content of the oxygen isotope ¹⁷O in the oxygen is selected to less than the naturally existing ratio, the content of the oxygen isotope ¹⁷O can be easily lowered to less than naturally existing ratio thereof by concentrating oxygen ¹⁶O at the naturally existing ratio of 99.76% as compared with the case of concentrating the oxygen isotope ¹⁷O at the naturally existing ratio of 0.037% and, accordingly, the production cost can be reduced remarkably.

Further, when a nuclear magnetic resonance agent at a low content of the oxygen isotope ¹⁷O is inhaled, the amount of H₂¹⁷O produced in cells is made smaller as compared with a case of inhaling usual air, and the relaxation time of the proton thereof is made longer than that of water containing H₂¹⁷O at the naturally existing ratio.

Accordingly, when signals for the proton is detected after the elapse of a predetermined period of time, for example, corresponding to the relaxation time of water having a natural composition, the signal intensity is increased for a normal portion since the amount of H₂¹⁷O produced is reduced to make the relaxation time longer whereas the amount of H₂¹⁷O does not change in the abnormal portion and, accordingly, the signal intensity is neither changed.

In this case, since tomographic images become bright in accordance with the signal intensity, it can be judged that the brightened portion is normal, whereas it can be judged that a portion not changing the brightness has abnormality.

In this case, although the content of H₂¹⁷O in the blood is reduced at a measuring site, since the blood is mixed during circulation in a body with a blood containing H₂¹⁷O at the naturally existing ratio, the content of H₂¹⁷O in the entire blood scarcely changes and, when the blood circulates, it gives no undesired effect on the detection of the signal intensity.

### (Brief Explanation of the Drawings)

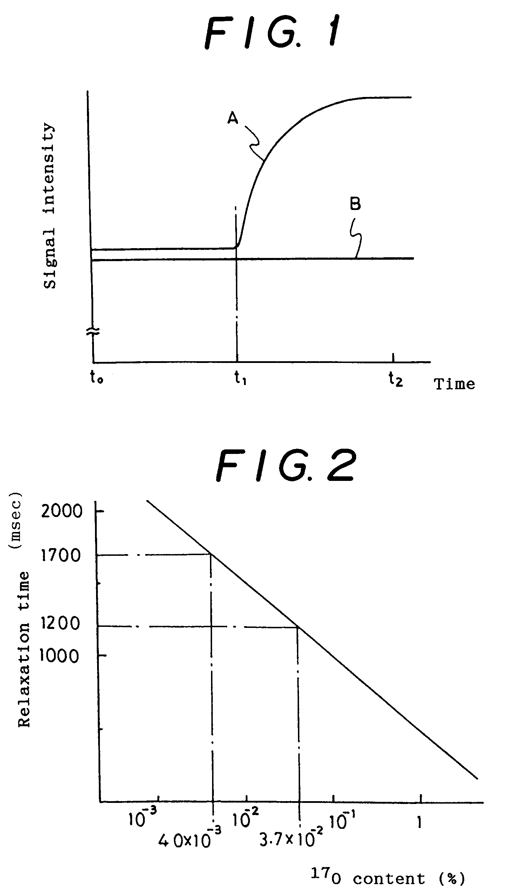

Fig. 1 is a graph illustrating the change of signal intensity detected by using the nuclear magnetic resonance agent according to the present invention, and Fig. 2 is a graph illustrating a relationship between relaxation time and concentration of ¹⁷O.

### (Best Mode for Practicing the Invention)

Description will now be made to the present invention based on more concrete examples.

In the nuclear magnetic resonance agent according to the present invention, oxygen at a concentration required for sustaining life is incorporated in an inhalation gas and the content of the oxygen isotope ¹⁷O in the oxygen is selected to less than the naturally existing ratio.

In the inhalation gas, an oxygen gas and a nitrogen gas are mixed, for example, at a ratio of 1:4 equal to the concentration of air, and the content of the oxygen isotope ¹⁷O in the oxygen gas is selected to less than the naturally existing ratio of 0.037 and, for example, oxygen contains 99.99% of ¹⁶O, 0.004% of ¹⁷O and 0.006% of ¹⁸O.

The inhalation gas is produced by concentrating ¹⁶O and, since the naturally existing ratio of ¹⁶O contained in the oxygen is originally as high as 99.76%, it can be concentrated relatively easily and the production cost can be suppressed to a reduced cost.

Since usual air in which an oxygen gas and a nitrogen gas are mixed at 1:4 ratio has been inhaled before measurement,oxygen contained in air is intaken through respiration into a lung.

The oxygen in air is bonded with hemoglobin in blood in the lung to form oxyhemoglobin which is circulated through a blood flow into a body to produce water by the metabolic function of cells.

Further, since the oxygen isotope ¹⁷O is contained by the naturally existing ratio in the oxygen, H₂¹⁷O is contained at a ratio equal with the naturally existing ratio also in the water produced by the metabolic function of the cells, and the production amount is maintained constant.

Then, when the nuclear magnetic resonance agent is inhaled, since the oxygen gas and the nitrogen gas are mixed at 1:4 ratio in the nuclear magnetic resonance agent and since the content of the oxygen isotope ¹⁷O in the oxygen gas is selected to about 0.004%, the content of H₂¹⁷O in the water produced by the metabolic function of the cells is also reduced.

Accordingly, when the proton of H₂O is detected along with time by NMR-CT before and after inhalation of the nuclear magnetic resonance agent, the content of H₂¹⁷O of the water produced by the metabolic function of cells is decreased to change the signal intensity.

For instance, Fig. 1 is a graph illustrating the change of signal intensity detected by using the nuclear magnetic resonance agent according to the present invention in which the ordinate indicates a signal intensity and the abscissa indicates time, and Fig. 2 is a graph illustrating a relationship between relaxation time and concentration of ¹⁷O in which the ordinate indicates relaxation time and the abscissa indicates ¹⁷O content by a logarithmic axis.

In Fig. 1, A shows signal intensity in a portion where the metabolic function is normal and B shows a signal intensity for a portion in which the metabolic function is abnormal.

At first, during inhalation of air, since oxygen in air is served for the metabolism, the oxygen concentration is kept constant and, at the same time, the concentration of ¹⁷O contained in the oxygen is kept at the naturally existing ratio.

When the change of the signal intensity is detected for the period (Fig. 1, t₀ - t₁), there is no change in the signal intensity both for the portion in which the metabolic function is normal and for the portion in which the metabolic function is abnormal since the composition of water present originally causes no change.

Then, when the supply of the nuclear magnetic resonance agent is started (Fig. 1, t₁), since the oxygen concentration in the nuclear magnetic resonance agent is equal with that in air, the concentration of oxyhemoglobin in the blood does not change by the supply of the nuclear magnetic resonance agent and the total amount of the water produced by the metabolism is also equal with that during inhalation of air.

However, in the nuclear magnetic resonance agent, since the content of the oxygen isotope ¹⁷O in the oxygen is selected to less than the naturally existing ratio, the amount of H₂¹⁷O produced by the metabolism is decreased.

Referring to the relationship between the content and the relaxation time of ¹⁷O in this case, as shown in Fig. 2, the relaxation time and the content are in a reverse proportion to each other on a semi-logarithmic graph and the relaxation time of water containing H₂¹⁷O, for example, at the naturally existing ratio (0.037%) is about 1200 msec, whereas the relaxation time of water containing H₂¹⁷O only by about 1/10 (0.004%) is about 1700 msec.

In the same manner, since the relaxation time is different for the water in the living tissue depending on the content of H₂¹⁷O, if detection is delayed for by a predetermined period of time delay in accordance with the relaxation time of water having a natural composition in the living tissue, the signal intensity for the normal portion is increased because the relaxation time of water at a reduced content of H₂¹⁷O produced in that portion is longer as compared with that of water having the natural composition.

Accordingly, if the resonance signals are detected along with time, the signal intensity increases gradually in a portion where the metabolism is normal (refer to Fig. 1A) (Fig. 1, t₁ - t₂) and the portion becomes brighter in tomographic images. On the other hand, in a portion in which the metabolism is failed (refer to Fig. 1B), since water is not produced originally water not produced by the metabolism but contained in cells is detected and, accordingly, the signal intensity does not change and a distinct contrast is formed between a normal portion and an abnormal portion.

The nuclear magnetic resonance agent may be supplied not only in a non-invasive manner by the inhalation of a gas as in the example but it may be supplied also in an invasive manner by utilizing a liquid capable of injecting into a living body and capable of carrying oxygen.

As such a liquid, a previously sampled blood or artificial blood may be considered, in which the content of the oxygen isotope ¹⁷O in the oxygen as the carrier may be selected to less than the naturally existing ratio.

Then, it may also be administrated, for example, through intravenous injection or injected into an artery flowing into a specific portion in a case of observing the metabolism at that portion.

Further, the nuclear magnetic resonance agent may be an inhalation gas containing carbon dioxide and oxygen at a concentration required for sustaining life in which the content of C¹⁷O₂ in the carbon dioxide is selected to less than the naturally existing ratio.

In this case, when carbon dioxide is intaken into a blood by respiration, ¹⁷O is replaced with ¹⁶O in H₂¹⁶O in the blood to produce H₂¹⁷O by the function of a carbonic anhydrase in the lung. When a nuclear magnetic resonance agent in which the content of C¹⁷O₂ in the carbon dioxide is selected to less than the naturally existing ratio is inhaled, the production amount is decreased and the signal intensity for the portion is increased if the metabolism in the lung is normal, so that information on the lung metabolism can be obtained.

Further, as the nuclear magnetic resonance agent, there may be used a first inhalation gas containing oxygen at least at a concentration required for sustaining life and a second inhalation gas to be supplied by an identical amount of supply with the first inhalation gas after the supply of the first inhalation gas for a predetermined period of time, in which the second inhalation gas is incorporated with oxygen at an identical concentration with the oxygen concentration in the first inhalation gas and the content of the oxygen isotope ¹⁷O in the oxygen is selected to less than the naturally existing ratio and to lower than the concentration of the oxygen isotope ¹⁷O contained in the first inhalation gas.

In this case, the first inhalation gas is at first inhaled for a predetermined period of time and, when the signal intensity is stabilized, the second inhalation gas is inhaled. At the same time, when change with time of the signal intensity is detected or tomography is conducted before and after the inhalation, the same detection result as in the previous example can be obtained.

Furthermore, this is not restricted only to the administration of oxygen and detection of water produced by the cell metabolism but may be injected in the form of water from the first into the living body.

In this case, water is used as a solvent for an injection solution to be injected into the living body and it is only necessary that the content of H₂¹⁷O in the water is selected to less than the naturally existent ratio (for example, 0.004%) and, for example, a physiological saline prepared by dissolving sodium chloride into water containing 0.004% of H₂¹⁷O may be used as the injection solution.

Then, in a case of observing the condition of a blood flow for a specified portion, when the nuclear magnetic agent is injected into a blood vessel which is imaged by NMR-CT, a portion in which the blood flows normally shines brightly since the content of H₂¹⁷O in water contained in the tissue is reduced by the nuclear magnetic resonance agent, whereas the signal intensity or the brightness does not change for a portion in which the blood does not flow due to coagulation or the like since the nuclear magnetic resonance agent does not flow.

Accordingly, the condition of the blood flow and absence or presence of the clogging or coagulation can be observed by imaging the nuclear magnetic resonance agent by NMR-CT.

### (Industrial Applicability)

As has been described above, the present invention can provide excellent effects capable of greatly reducing the production cost giving no undesired effects on signal detection, and, at the same time, if the blood is circulated in the body after a great amount of administration, since there is no requirement for using oxygen prepared by concentrating ¹⁷O, obtaining, accurate information on the metabolic function by detecting the signal intensity since the signal intensity is high for a normal portion and the portion becomes bright upon tomography, whereas the signal intensity does not change for an abnormal portion and no change is observed upon tomography.

## Claims

1. A nuclear magnetic resonance agent wherein an inhalation gas is incorporated with oxygen at a concentration required for sustaining life and the content of an oxygen isotope ¹⁷O in the oxygen is selected to less than the naturally existing ratio.

2. A nuclear magnetic resonance agent wherein oxygen is carried on a liquid capable of injecting into a living body and capable of carrying oxygen, and the content of oxygen isotope ¹⁷O in the oxygen is selected to less than the naturally existing ratio.

3. A nuclear magnetic resonance agent wherein water is used as a solvent for an injection solution to be injected into a living body and the content of H₂¹⁷O in the water is selected to less than the naturally existing ratio.

4. A nuclear magnetic resonance agent wherein carbon dioxide and oxygen at a concentration required for sustaining life are incorporated and the content of C¹⁷O₂ in the carbon dioxide is selected to less than the naturally existing ratio.

5. A nuclear magnetic resonance agent which is used upon causing hydrogen H of H₂O present in the living body to nuclear magnetic resonance and detecting the change with time of resonance signals thereof, wherein the magnetic resonance agent comprises a first inhalation gas incorporated with oxygen at least at a concentration required for sustaining life and a second inhalation gas to be supplied at an identical amount of supply with the first inhalation gas after the supply of the first inhalation gas for a predetermined period of time, the second inhalation gas is incorporated with oxygen at an identical concentration with the oxygen concentration in the first inhalation gas, and the content of oxygen isotope ¹⁷O in the oxygen is selected to less than the naturally existing ratio and to lower than the concentration of the oxygen isotope ¹⁷O contained in the first inhalation gas.
